# EUROPEAN PATENT APPLICATION

(11) **EP 4 282 439 A1**
(43) Date of publication of application: **29.11.2023**
(21) Application number: 22174933.6
(22) Date of filing: 23.05.2022
(51) Int. Cl.: A61K 51/08, A61K 103/00

(54) **RADIOISOTOPE LABELED SSTR2-AGONISTS WITH LINKERS**

(71) Applicant: Erasmus University Rotterdam Medical Center, 3015 GE Rotterdam (NL)
(72) Inventor: SEIMBILLE, Yann, 3015 GE Rotterdam (NL); CHAPEAU, Dylan, 3015 GE Rotterdam (NL)
(74) Representative: V.O.

(57) **Abstract**

The invention is directed to a pharmaceutical compound, or a pharmaceutically acceptable salt thereof, for use in a medical treatment or diagnosis of tumors, in particular neuroendocrine tumors (NET). The compound is according to the formula Ch(M)-L-T, wherein Ch represents a radioisotope chelator; M represents the radioisotope; T represents a sstr2-agonist; L represents a linker comprising a moiety having a six-membered cyclic structure.

## Description

The invention is in the field of nuclear medicine, more particularly in the field of radiopharmaceuticals that can be used for the imaging and treatment of neuroendocrine tumors (NETs).

Neuroendocrine tumor (NET) is a rare type of tumor, which occurs with an incidence of approximately 4 per 100'000 individuals. The majority of NETs is slow-growing and relatively asymptomatic until the tumor spreads. Systemic treatments are often necessary since most of the patients (40-95%) are presenting evidence of metastatic spread at diagnosis. Treatment of NETs depends on many factors due to their heterogeneity, such as location, aggressiveness, or intrinsic biology.

On NET cells, somatostatin receptors and especially the subtype 2 thereof (sstr2), are widely overexpressed. The high prevalence of sstr2 expression in NET cells has led to the development of sstr2-agonists, such as octreotide and octreotate, which can be labelled with indium-111 (gamma emitter) or gallium-68 (positron emitter) for SPECT and PET imaging, respectively, or with beta-emitters (⁹⁰Y and ¹⁷⁷Lu) and alpha-emitters (²²⁵Ac and ²¹²Pb) for therapeutic purposes. See for instance Breeman et al. Seminars in Nuclear Medicine, 2011, 41(4):314-321 (and references therein) for ⁶⁸Ga-labeled analogs of octreotide, such as DOTA-TOC, DOTA-NOC, and DOTA-TATE and their clinical application in nuclear medicine. Rozgaja Stallons et al., Mol. Cancer Ther. 2019, 18:1012-1021 disclose preclinical investigation of ²¹²Pb-DOTAMTATE for peptide receptor radionuclide therapy in a neuroendocrine tumor model.

The labeling of sstr2-agonist such as octreotide and octreotate (abbreviated as TATE) with radioisotopes, is achieved by coupling the agonist to a chelator that is capable to strongly bind the radioisotope. A drawback of this concept, however, is that chelator may negatively influence the binding of the agonist to the receptor. This can lead to a reduced tumor uptake and higher accumulation in off-target tissues of the radioisotope.

The clinical results of peptide receptor radionuclide therapy (PRRT) based on sstr2-agonists are encouraging, but the overall response rate after treatment with [¹⁷⁷Lu]Lu-DOTA-octreotate ([¹⁷⁷Lu]Lu-DOTATATE) are still insufficient. The maximum injected dose of the sstr2-radioligand is actually limited by the maximum acceptable absorbed dose to nontarget organs (i.e. 2 Gy for the bone marrow and approximately 40 Gy for the kidneys). Unfortunately, this injected dose is not enough to completely eradicate the tumor, and disease stabilization is observed in only a part of all treated patients.

Attempts to improve the overall response rate have been made by positioning linkers (which can be regarded as spacers) in between the agonist and the chelator.

WO2018/132751 discloses compounds wherein a chelator with a radioisotope is linked via a linker to octreotate, such as ²⁰³Pb-TCMCTATE.

WO2021/154921 discloses compounds wherein a chelator with a radioisotope is linked via a polyethylene glycol linker to Tyr³-octreotide (TOC), such as ^{203/212}Pb-PSC-PEG₂-TOC.

Thus far however, the compounds with linkers however, have not led to an overall improvement and the clinically used compounds remain the unlinked variants, such as DOTATATE or DOTAMTATE.

Accordingly, the need to provide improved radiopharmaceuticals that can be used for the imaging and treatment of neuroendocrine tumors remains. In particular, there is a need to provide radiopharmaceuticals that give higher tumor uptake and lower accumulation in off-target tissues, leading to a better therapeutic index.

The present inventors found a type of linker that surprisingly results in higher tumor uptake and lower accumulation in off-target tissues compared to the clinical reference compounds.

Accordingly, an aspect of the present inventions is directed to a pharmaceutical compound, or a pharmaceutically acceptable salt thereof, according to formula (I)

Ch(M)-L-T (I)

wherein Ch represents a radioisotope chelator;
M represents the radioisotope;
T represents a sstr2-agonist;
L represents a linker comprising a moiety having a six-membered cyclic structure..

### Linker

Without wishing to be bound by theory, the six-membered cyclic structure of the moiety that is part of the linker may provide rigidity to the linker which is beneficial for good binding of the sstr2-agonist to its cognate receptor.

The linker preferably has a structure according to any of formulae IIa and IIb 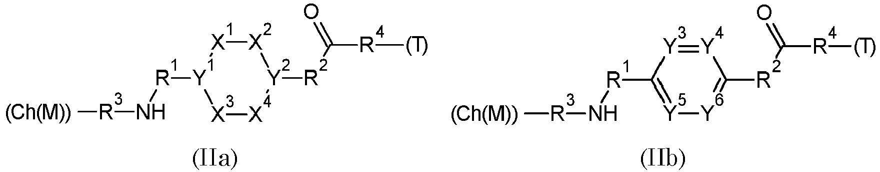 wherein
X¹-X⁴ are independently selected from C, O and N, optionally substituted by one or more heteroatoms;
Y¹-Y⁶ are independently selected from N and C, optionally substituted by one or more heteroatoms;

R¹ and R² are independently selected from the group consisting of a bond hydrocarbylenes, preferably branched and linear (C₁-C₁₀)-alkylenes (*i.e*. alkanediyls), which are optionally substituted and/or interrupted by one or more heteroatoms; and
R³ and R⁴ represent an optionally present spacer.

One or more spacers are optionally present in the linker. Accordingly, R³ and R⁴ are individually selected from the groups consisting of a bond and spacers. The spacers are preferably aliphatic. Suitable spacers may be based on linear or branched amino acids such as glycine, alanine, β-alanine, 3-aminopropionic acid, 4-aminobutyric acid, 5-aminovaleric acid, 6-aminohexanoic acid, 7-aminoheptanoic acid, 8-aminooctanoic acid, 9-aminononanoic acid, 10-aminodecanoic acid, 2-aminooctanoic acid, and the like. In some embodiments, the spacer is a peptide spacer (Xaa)₁₋₄, wherein each Xaa is independently a proteinogenic or non-proteinogenic amino acid residue. Herein, each peptide backbone amino group may be independently optionally methylated. In particular embodiments, each non-proteinogenic amino acid residue is independently selected from the group consisting of a D-amino acid of a proteinogenic amino acid, *N^{ε}*,*N^{ε}*,*N^{ε}*-trimethyl-lysine, 2,3-diaminopropionic acid (Dap), 2,4-diaminobutyric acid (Dab), ornithine (Orn), homoarginine (hArg), 2-amino-4-guanidinobutyric acid (Agb), 2-amino-3-guanidinopropionic acid (Agp), β-alanine, 4-aminobutyric acid, 5-aminovaleric acid, 6-aminohexanoic acid, 7-aminoheptanoic acid, 8-aminooctanoic acid, 9-aminononanoic acid, 10-aminodecanoic acid, 2-aminooctanoic acid, 2-aminoadipic acid ( 2-Aad), 3-aminoadipic acid (3-Aad), cysteic acid, diglycolic acid, NH₂(CH₂)₂O(CH₂)₂C(O)OH, NH₂(CH₂)₂[O(CH₂)₂]₂C(O)OH (dPEG2), NH₂(CH₂)₂[O(CH₂)₂]₃C(O)OH, and NH₂(CH₂)₂[O(CH₂)₂]₄C(O)OH.

As used herein, if a moiety or group can be selected from a group comprising a 'bond', this effectively means that this moiety or group is absent from the structure and that the directly adjacent moieties or groups are directly bound to each other.

As used herein, heteroatoms that may be substituents or may interrupt moieties such as alkylene and alkyl groups as disclosed herein, include O, NH₂, SO₂, and halogens such as F, Cl, Br and I. It may be appreciated that for any moiety that may optionally be substituted and/or interrupted with one or more heteroatoms, such a moiety may also be unsubstituted and/or uninterrupted by said heteroatoms.

For any formulae of a structure that is part of a compound disclosed herein, the relative position of said structure vis-à-vis other parts of the compound is indicated by indicating the other between brackets. For instance, in formulae IIa and IIb, the relative position of the chelator and the radioisotope is indicated with (Ch(M)), while that of the sstr2-agonist is indicated with (T).

Preferably, one of R¹ and R² is a bond, while the other is selected from the groups consisting of branched and linear (C₁-C₁₀)-alkylenes, preferably branched and linear (C₁-C₄)-alkylenes, most preferably methylene (-CH₂-). In embodiments wherein R¹ is a bond and R² is selected from the groups consisting of branched and linear (C₁-C₁₀)-alkylenes, Y² is preferably N or CH.

In a preferred embodiment, the linker L has a structure according to any of formulae IIaa, Iiab, Iiba and Iibb 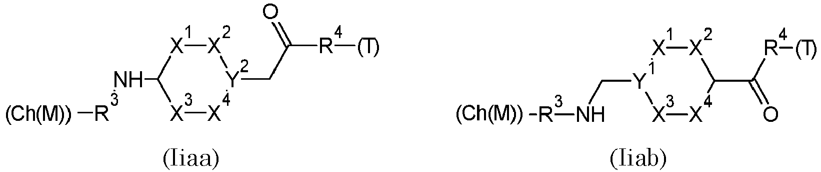 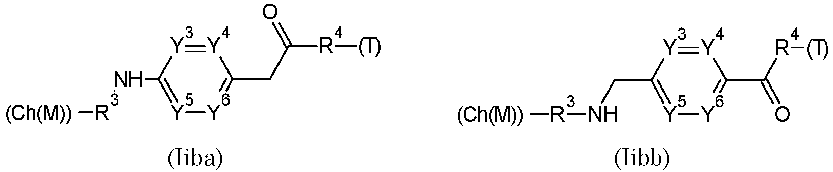 wherein X¹-X⁴, Y¹-Y⁶ and R³ and R⁴ are as defined for formula Iia and lib.

In a preferred embodiment, for formulae Iia, Iiaa and Iiab, one of X¹-X⁴ and Y¹-Y² is selected from the O and N and the others are C, which may optionally be substituted by one or more heteroatoms. For formulae IIb, Iiba and Iibb, preferably one of Y³-Y⁶ is selected from C and N and the others are C, which may optionally be substituted by one or more heteroatoms. As such, the six-membered ring contains one heteroatom.

In yet another embodiment, all of X¹-X⁴ and Y¹-Y⁶ are C, which may optionally be substituted by one or more heteroatoms.

It may be appreciated that when a group or moiety is said to be selected from a group containing C or N, this C or N has, in a non-fully substituted state, the required number of hydrogen atoms to fulfill the octet role.

In a further preferred embodiment, the linker L has a structure according to any of formulae IIc-IIl 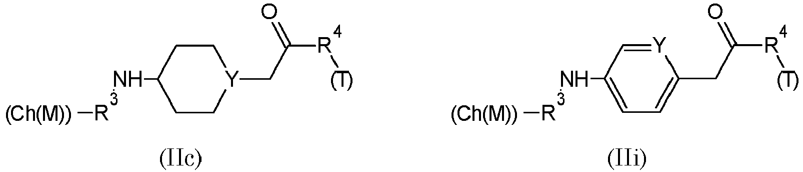 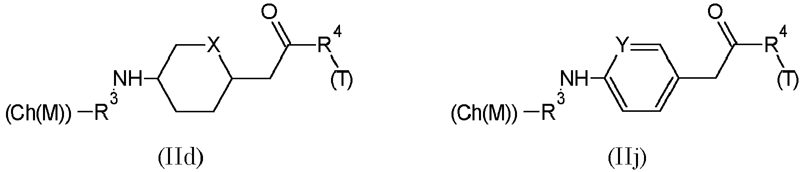 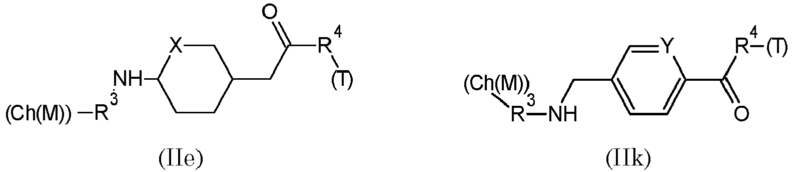 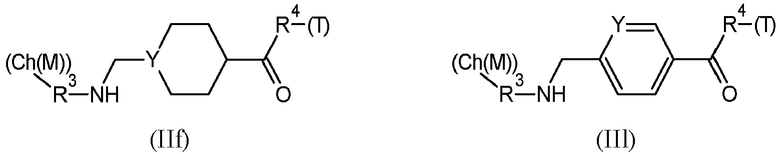 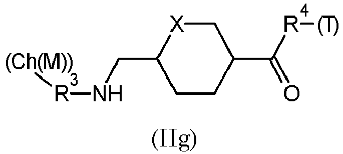 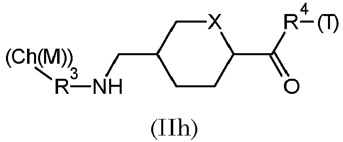 wherein
R³ and R⁴ are as defined for formulae IIaa, IIab, IIba and IIbb; and
X is selected from the group consisting of C, N, and O, preferably N and C; more preferably C;
Y is selected from the group consisting of C and N, preferably N for formula IIc and C for formula IIi-IIl.

Particularly good results were obtained with aliphatic linkers, which are therefore preferred. Accordingly, the linker with a structure according to any of formulae IIc-IIh is preferred

In a yet further preferred embodiment, the linker L has a structure according to any of formulae IIca and IIfa, which are specific versions of formulae IIc and IIf respectively, 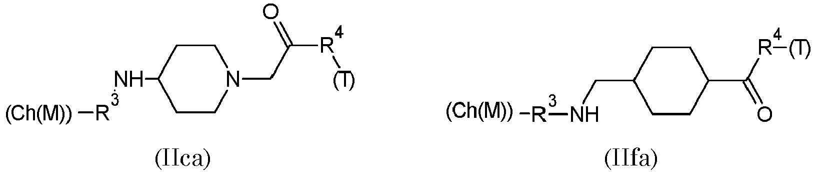 wherein R³ and R⁴ are as defined for formulae IIa, IIb, IIaa, IIab, IIba and IIbb.

Unless the stereochemistry of an atom has been specifically indicated herein, the stereochemistry of said atom is undefined which indicated that said structure represents all possible stereoisomers. For instance, structure IIfa represents at least two diastereoisomers: the *trans* and *cis*-isomers. However, preferably, the linker represented by this formula has the trans-configuration, as indicated by formula IIfa' below.

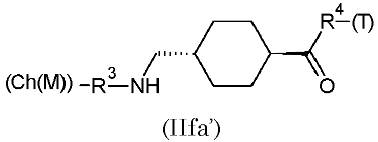

The linkers having the structures according to formulae IIca and IIfa are based on 4-amino-1-carboxymethyl-piperidinyl (Pip) and 4-(aminomethyl)cyclohexane-1-carbonyl (Amcha), respectively. The Pip-containing linker, like any other amine-group containing linker disclose herein, may be cationic under physiological conditions, and as such could affect the global charge of the conjugate.

The Pip- and Amcha-based linkers as disclosed herein are preferred. Particularly good results were obtained with the linkers without any additional spacers. Accordingly, the linker L has a structure according to any of formulae IIm and IIn.

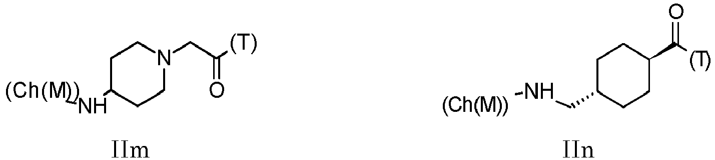

Most preferably, the linker has a structure according to formula IIn.

The sstr2-agonist for the present invention can be any compound or ligand that is capable of binding to sstr2. Such agonists are known in the art. Typically, the sstr2-agonist is a peptide, an antibody, an antibody fragment or a small molecule. In preferred embodiments, the sstr2-agonist is an octreotide or a derivative thereof. In this context, derivatives mean that one or more amino acids residues may be substituted or modified. Examples of known derivates, which are also suitable for the present invention, include Tyr³-octreotide (TOC), Tyr³-octreotate (TATE) and 1-Nal³-octreotide (NOC, wherein 1-Nal is 1-naphthylalanine). In a preferred embodiment, the sstr2-agonist is selected from the group consisting of octreotide and octreotate, most preferably the sstr2-agonist is octreotate, or a pharmaceutically acceptable salt thereof.

The radioisotope chelator (also referred to as chelator) can suitably be coupled via the linker to the amine group of the terminal phenylaniline (¹Phe) of the octreotide or the derivative thereof. However, other positions may also be suitable, as long as this is not detrimental to the binding with sstr2. Accordingly, in a preferred embodiment, the sstr2-agonist T has a structure according to formula (III), 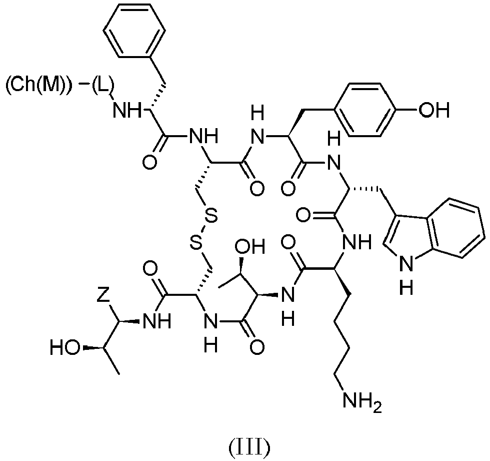 wherein Z is selected from the groups consisting of -CO₂H and -CH₂-OH.

### Chelator

In the field, various chelators are known to bind radioisotopes. The chelator for the present invention is a pharmaceutically acceptable chelator. The chelator is typically selected based on the radioisotope that is desired for the diagnosis or treatment as not all chelators are equally suitable for all radioisotopes. Typical the radioisotope chelator Ch comprises a cyclic or branched polyaminopolycarboxylic moiety or amide derivative thereof. See for instance Tornesello et al., Molecules 2017, 22(8), 1282, US2021/0402016A1, WO2021/005125A1 and Price and Orvig, Chem. Soc. Rev., 2014, 43, 260-290 and references cited therein.

In preferred embodiments of the present invention, the radioisotope chelator Ch is based on compounds selected from the group consisting of
- DOTA (1,4,7, 10-tetraazacyclodocecane-N,N',N",N"'-tetraacetic acid, also known as tetraxetan) and derivatives such as p-SCN-Bn-DOTA (2-(4-isothiocyanatobenzyl)-1,4,7,10-tetraazacyclododecane tetraacetic acid);
- PSC (1,4,7,10-tetraazacyclododecane-7-acetamide-1,4,10-triacetic acid);
- DO3A (1,4,7,10-tetraazacyclodocecane-N,N',N"-triacetic acid);
- DOTAGA (1,4,7,10-tetraazacyclododececane, 1-(glutaric acid)-4, 7,10-triacetic acid);
- DO3AM (2,2',2"-(1,4,7,10-tetraazacyclododecane-1,4,7-triyl)triacetamide);
- DOTAM (2-[4,7,10-tris(2-amino-2-oxoethyl)-1,4,7,10-tetrazacyclododec-1-yl]acetamide) and derivatives such as p-SCN-Bn-TCMC (2-(4-isothiocyanatobenzyl)-1,4,7,10-tetraaza-1,4,7,10-tetra(2-carbamoylmethyl)cyclododecane);
- NOTA (1,4,7-triazacyclononane-N,N',N"-triacetic acid);
- NODAGA (1-(1,3-carboxypropyl)-4,7-carboxymethyl-1,4,7-triazacyclononane);
- NODASA (1,4,7-triazacyclononane-1-succinic acid-4,7-diacetic acid);
- CB-DO2A (4,10-bis(carboxymethyl)-1,4,7,10-tetraazabicyclo[5.5.2]tetradecane);
- 3p-C-DEPA (2-[(carboxymethyl)][5-(4-nitrophenyl-1-[4,7,10-tris-(carboxymethyl)-1,4,7,10-tetraazacyclododecan-1-yl]pentan-2-yl)-amino] acetic acid);
- TCMC (1,4,7,10-tetrakis(carbamoylmethyl)1,4,7,10-tetraazacyclododecane);
- DTPA (diethylenetriaminepentaacetic acid) and DTPA derivatives such as CHX-A"-DTPA (2-(p-isothiocyanatobenzyl)cyclohexyldiethylenetriaminepentaacetic acid and 1B4M-DTPA;
- TETA (1,4,8,11-tetraazacyclotetradecane 1,4,8,11-tetraacetic acid) and analogues and derivatives such as C-NETA;
- NE3TA ((7-[2-[carboxymethyl)amino]ethyl]-1,4,7-triazacyclononane-1,4-diacetic acid) and derivatives such as C-NE3TA;
- CB-TE2A (4,11-bis(carboxymethyl)-1,4,8,11 tetraazabicyclo[6.6.2]hexadecane);
- NETA ({4-[2-(bis-carboxymethylamino)-ethyl]7-carboxymethyl-[1,4,7]triazonan-1-yl)-acetic acid); and NETA derivatives such as 3p-C-NETA (see Sun et al. ACS Omega 2020, 5, 44, 28615-28620)
- H₂azapa (N,N'-[1-benzyl-1,2,3-triazole-4-yl]methyl-N,N'-[6-(carboxy)pyridin-2-yl)-1,2-diaminoethane) and other picolinic acid derivatives such as H₂dedpa (1,2-[6-(carboxy)-pyridin-2-yl)methylamino)ethane, H₄octapa (N,N'-bis(6-carboxy-2-pyridylmethyl)-
- ethylenediamine-N,N'-diacetic acid), H₄py4pa, H₄Pypa, H₆phospha, H₄CHXoctapa, H₅decapa (N,N"-[[6-(carboxy)pyridin-2-yl]methyl]-
- diethylenetriamine-N,N',N"-triacetic acid), and H₄neunpa-p-Bn-NO₂;
- SHBED (N,N'-bis(2-hydroxy-5-sulfobenzyl)ethylenediamine-N,N'-diacetic acid);
- HBED (N,N'-bis(2-hydroxybenzyl)-ethylenediamine-N,N'-diacetic acid);
- H2-MACROPA (N,N'-bis[(6-carboxy-2-pyridil)methyl]-4,13-diaza-18-crown-6);
- PCTA (3,6,9,15-tetraazabicyclo[9.3.1]pentadeca-1(15),11,13-triene-3,6,9-triacetic acid);
- Me-3,2-HOPO (see Ramdahl, Bioorganic & Medicinal Chemistry Letters 26 (2016) 17, 4318-4321);
- CB-TE1A1P (1,4,8,11-tetraazacyclotetradecane-1-(methanephosphonic acid)-8-(methanecarboxylic acid));
- CB-TE2P (1,4,8,11-tetraazacyclotetradecane-1,8-di(methanephosphonic acid);
- MM-TE2A (N-monomethyl 1,8-N,N'-bis-(carboxymethyl)-1,4,8,11-tetraazacyclotetradecane);
- DM-TE2A (N,N'-dimethyl 1,8-N,N'-bis-(carboxymethyl)-1,4,8,11-tetraazacyclotetradecane);
- sarcophagine and sarcophagine derivatives such as SarAr (1-N-(4-aminobenzyl)-3,6,10,13,16,19-hexaazableyelo[6.6.6]-elcosane-1,8-diamine, diamSar, AmBaSar, and BaBaSar;
- TRAP (1,4,7-triazacyclononane-1,4,7-tris[methyl(2-carboxyethyl)phosphinic acid) and analogues such as NOPO (1,4,7-triazacyclononane-1,4-bis[methylene(hydroxymethyl)phosphinic acid]-7-[methylene(2-carboxyethyl)phosphinic acid]);
- AAZTA (1,4-bis(hydroxycarbonyl methyl)-6-[bis(hydroxylcarbonylmethyl)] amino-6-methyl perhydro-1,4-diazepine);
- DATA and DATA derivatives;
- CP256 (4-acetamido-N1,N7-bis-[(3-hydroxy-1,6-dimethyl-4-methylene-1,4-dihydropyridin-2-yl)methyl]-4-(3-[(3-hydroxy-1,6-dimethyl-4-methylene-1,4-dihydropyridin-2-yl)methylamino]-3-oxopropyl)heptanediami) and its derivative YM103 (4-(3-[3-(2,5-dioxo-2,5-dihydro-1H -pyrrol-1-yl)propanamido]propanamido)-N1,N7-bis[(3-hydroxy-1,6-dimethyl-4-methylene-1,4-dihydropyridin-2-yl)methyl]-4-(3-[(3-hydroxy-1,6-dimethyl-4-methylene-1,4-dihydropyridin-2-yl)methylamino]-3-oxopropyl)heptanediamide);
- PCTA (6,9,15-tetraazabicyclo[9.3.1]pentadeca-1(15),11,13-triene-3,6,9,-triacetic acid);
- BCPA (see Price and Orvig, Chem. Soc. Rev., 2014, 43, 260-290);
- DFO (desferrioxamine) and DFO derivatives ;
- a trithiol chelate;
- mercaptoacetyl;
- hydrazinonicotinamide;
- dimercaptosuccinic acid;
- 1,2-ethylenediylbisL-cysteine diethyl ester;
- methylenediphosphonate;
- hexamethylpropyleneamineoxime;
- hexakis(methoxy isobutyl isonitrile);
and analogues thereof.

Most preferred are the DOTA and DOTAM chelators as these chelate particularly well to ¹⁷⁷Lu and ²¹²Pb, respectively.

Preferably, the chelator is bound to the linker via one of the amide groups or one of the carboxylic acid groups of the chelator, leading to an amide bond. However, it may also be bound via one of its carbon atoms. For linkage to a carbon atom, the chelator may be appropriately equipped with an isothiocyanate functionality, for instance an isothiocyanatobenzyl. Examples of such equipped chelators include pSCN-Bn-DOTA and p-SCN-Bn-TCMC.

In preferred embodiments, the linker is bound to one of the carboxylic acid groups of the chelator.

Accordingly, the radioisotope chelator Ch in particular embodiments has a structure according to any of formula IVa-IVf, preferably IVa or IVc, more preferably IVa 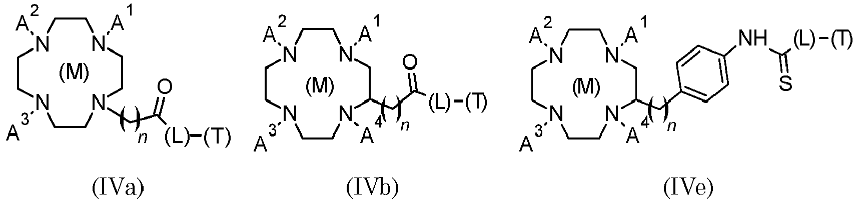 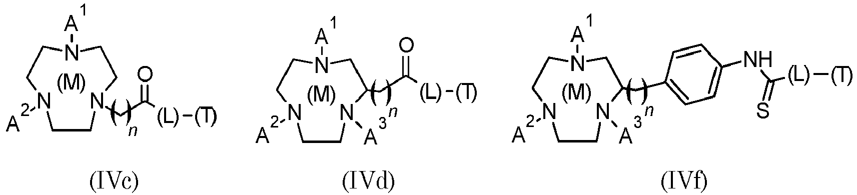 wherein *n* is 1-6, preferably 1; and

A¹-A⁴ are independently selected from the group consisting of H, alkyls, aliphatic acids, such as carboxylic acids, and amides and esters thereof. Preferably, A¹, A² and A³ are independently selected from the group consisting of H, (C₁-C₆)-alkyl, (C₁-C₆)-alkylene-(CO₂A⁵), (C₁-C₆)-alkylene-(C(O)NA⁵A⁶), wherein A⁵ and A⁶ are independently selected from the group consisting of H and (C₁-C₆)-alkyls, preferably H. In a further preferred embodiment, A¹-A⁴ are all the same and selected from the group consisting of -CH₂CO₂H and -CH₂C(O)NH₂.

### Radioisotope

The radioisotope may be any suitable radioisotope. See for instance Tornesello et al., Molecules 2017, 22(8), 1282, US 2021/0402016A1, WO2021/005125A1 and Price and Orvig, Chem. Soc. Rev., 2014, 43, 260-290 and references cited therein. The radioisotope M may be a α- or β-particle emitter, a positron emitter and/or a γ-emitter. The α-particle emitters and β-particle emitters, such as ⁹⁰Y, ²¹²Pb, ¹⁷⁷Lu, ¹⁸⁸Re, ¹⁸⁶Re, ⁶⁷Cu, ⁶⁴Cu, ²¹²Bi, ²¹³Bi, ²¹¹At, ²²⁵Ac, ¹³¹I and the like can be used for therapy. Suitable γ-emitters such as ^{99m}Tc, ⁶⁷Ga, ¹¹¹In and the like can be used for SPECT imaging, while positron emitters such as ⁶⁸Ga, ⁶⁴Cu,¹⁸F and the like are useful for PET imaging. Accordingly, the radioisotope M for the present invention is preferably is selected from the group consisting of ²¹²Pb, ²⁰³Pb, ⁶⁴Cu, ⁶⁷Cu, ²¹²Bi, ⁶⁸Ga, ²¹³Bi, ²²⁵Ac, ²⁴³Am, ²¹¹At, ²¹⁷At, ¹⁵⁴Dy, ¹⁴⁸Gd, ¹⁴⁶Sm, ¹⁴⁷Sm, ¹⁴⁹Tb, ¹⁵²Tb, ¹⁵⁵Tb, ¹⁶¹Tb, ¹⁶⁵Er, ⁷²As, ⁷⁷As, ⁴⁷Sc, ¹⁸⁸Re, ¹⁸⁶Re, ¹⁰⁵Rh, ¹⁰⁹Pd, ¹⁹⁹Au, ¹⁷⁵Yb, ¹⁴²Pr, ^{114*m*}In, ^{94*m*}Tc, ^{99m}Tc, ²²⁷Th, ²²⁹Th, ⁵⁹Fe, ⁶⁰Cu, ⁶¹Cu, ⁶²Cu, ⁶⁷Ga, ⁴⁴Sc, ⁸⁹Zr, ⁹⁰Nb, ⁸⁶Y, ⁹⁰Y, ¹¹¹In, ¹⁷⁷Lu, ^{117*m*}Sn, ¹⁵³Gd, ¹⁵³Sm, and ¹⁶⁶Ho, preferably from the group consisting of ⁶⁴Cu, ⁶⁷Ga, ⁶⁸Ga, ⁹⁰Y, ¹¹¹In, ¹⁷⁷Lu, ²¹²Pb and ²²⁵Ac, most preferably from the group consisting of ²¹²Pb and ¹⁷⁷Lu.

### Exemplary compounds

In a particularly preferred embodiment, the pharmaceutical compound, or a pharmaceutically acceptable salt thereof, has a structure according to any of formulae Ia and Ib, preferably according to formula Ia, wherein Ch, M and Z are as defined above. 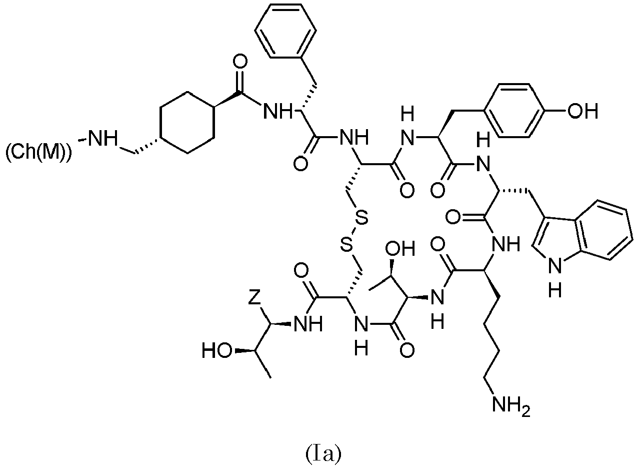 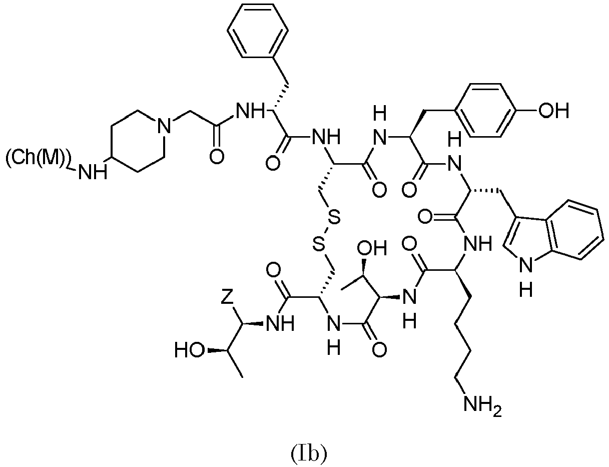 wherein Ch, M and Z are as defined in any of the previous claims.

In a further preferred embodiment, the compound is of formula Ia, wherein Z is -CO₂H (such that the sstr2-agonist is TATE), Ch is DOTAM and M is ²¹²Pb. This embodiment can be abbreviated as [2¹²Pb]Pb-DOTAM-Amcha-TATE.

Another particularly preferred embodiment, the compound is of formula Ia, wherein Z is -CO₂H (such that the sstr2-agonist is TATE), Ch is DOTA and M is ¹⁷⁷Lu. This embodiment can be abbreviated as [¹⁷⁷Lu]Lu-DOTA-Ameha-TATE.

The compound or a pharmaceutically acceptable salt thereof, can be for use as a medicament. More specifically, for use in a medical treatment or diagnosis of tumors, in particular neuroendocrine tumors (NETs). Whether it can be used in treatment or diagnosis mostly depends on the used radioisotope, as described herein.

Accordingly, a further aspect of the present invention is directed to a method for the treatment or diagnosis of tumors, in particular neuroendocrine tumors (NETs). Said method comprises administration of the compound or a pharmaceutically acceptable salt thereof to a patient in a pharmaceutically acceptable dose.

As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features.

For the purpose of clarity and a concise description features are described herein as part of the same or separate embodiments, however, it will be appreciated that the scope of the invention may include embodiments having combinations of all or some of the features described.

The present invention can be illustrated by the following non-limited embodiments.

### General

- HPLC analyses of the reactions were performed using an analytical Phenomenex (Torrance, CA, USA) Gemini RP-C18 column (5 pm, 250 × 4.60 mm) at a flow rate of 1 mL/min. The UV signal was recorded at 280 nm. The following solvents and eluting conditions were used:
   Solvent A: 0.1% Trifluoroacetic acid (TFA) in water (v/v).
   Solvent B: 0.1% TFA in acetonitrile (v/v).

The following gradient of solvents A and B was applied: 0-3 min, 5% B; 3-23 min, 5-100% B; 23-27 min, 100% B
- HPLC purifications were performed with a semi-preparative Phenomenex Luna RP-C18 column (10 pm, 250 × 10 mm) at a flow rate of 3 mL/min. The UV signal was recorded at 280 nm. The following solvents and eluting conditions were used:
   Solvent A: 0.1% Trifluoroacetic acid (TFA) in water (v/v).
   Solvent B: 0.1% TFA in acetonitrile (v/v).
   **Condition 1:** 0-2 min, 10% B; 2-3 min, 10-25% B 3-23 min, 25% B; 23-27 min, 25-90% B.
   **Condition 2:** 0-23 min, 15-30% B; 23-28 min, 30-90% B; 28-33 min, 90% B.
- UPLC analyses for titration were performed using an ACQUITY UPLC (Torrance, CA, USA) HSS C18 column (18 pm, 50 × 2.10 mm) at a flow rate of 0.5 mL/min. The UV signal was recorded at 280 nm. The following solvents and eluting conditions were used:
   Solvent A: 0.1% Trifluoroacetic acid (TFA) in water (v/v).
   Solvent B: 0.1% TFA in acetonitrile (v/v).

The following gradient of solvents A and B was applied: 0-0.2 min, 5-25% B; 0.2-2.5 min, 25% B; 2.5-2.8 min, 25-100% B; 2.8-2.9 min.

### Example 1-preparation of D-Phe-cyclo[Cys-Tyr(tBu)-D-Trp(Boc)-Lys(Boc)-Thr(tBu)-Cys]-Thr(tBu)-resin (1)

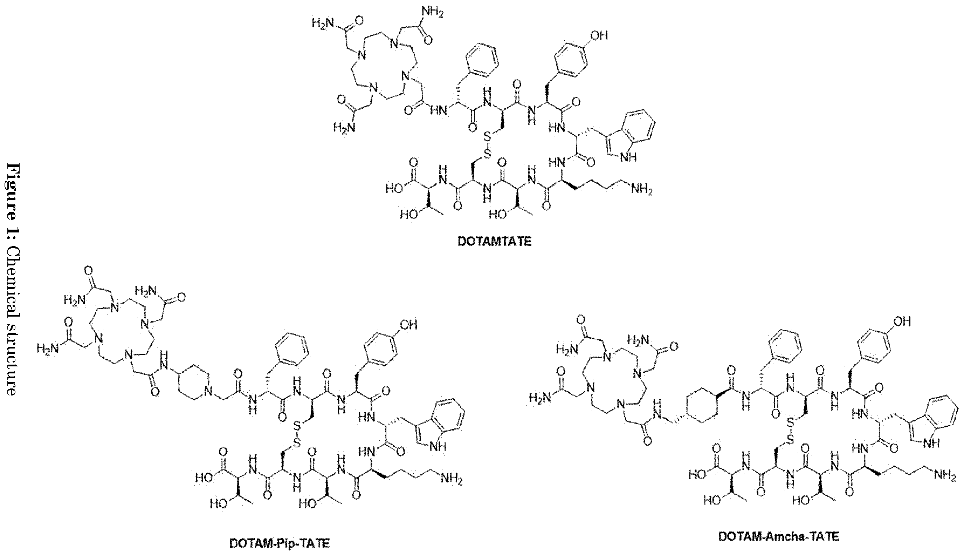

D-Phe-Cys(Acm)-Tyr(*t*Bu)-D-Trp(Boc)-Lys(Boc)-Thr(*t*Bu)-Cys(Acm)-Thr(*t*Bu)-resin was synthesized using a *N^{α}*-Fmoc solid-phase peptide synthesis strategy. The conjugation of Fmoc protected amino acid (4.0 equiv.) to the 2-chlorotrityl chloride resin was carried out in dimethylformamide (DMF) using hexafluorophosphate azabenzotriazole tetramethyl uronium (HATU) (3.8 equiv.) and *N*,*N*-diisopropylethylamine (DIPEA) (7.8 equiv.) for 45 minutes. Fmoc deprotection was accomplished by treatment of the resin with a 20% solution of 4-methylpiperidine in DMF. Amide formation and Fmoc deprotection were monitored by TNBS test. Coupling or Fmoc deprotection were performed twice when the reaction was not completed. The peptide synthesis was started by loading Fmoc-L-Thr(*t*Bu)-OH onto the 2-chlorotrityl chloride resin (0.25 g, average loading capacity: 1.6 mmol/g). The resin was shaken for 90 min at room temperature (rt). The resin was capped using 8 mL of dichloromethane (DCM)/MeOH/DIPEA (v/v/v = 80:15:5) for 15 min at rt. Subsequent Fmoc deprotection and coupling with Fmoc-L-Cys(Acm)-OH, Fmoc-L-Thr(*t*Bu)-OH, Fmoc-L-Lys(Boc)-OH, Fmoc-D-Trp(Boc)-OH, Fmoc-L-Tyr(*t*Bu)-OH, Fmoc-L-Cys(Acm)-OH and Fmoc-D-Phe-OH were achieved following the same protocol described above. After the ultimate Fmoc deprotection, the protected linear peptide was cyclized on resin by using Tl(TFA)₃ (2.0 equiv.) in DMF for 1 h at room temperature. The cyclization was monitored by analytical HPLC. The structure of (1) is given in Figure 1.

### Example 2 - preparation of DOTAM-(D-Phe-cyclo[Cys-Tyr-D-Trp-Lys-Thr-Cys]-Thr-OH (DOTAMTATE) (2)

A fraction of the cyclic peptidyl resin 1 (40 µmol), prepared according to Example 1, was swollen in DMF. DO3AM acetic acid (50 mg, 120 µmol, 3 equiv.), PyBop (65 mg, 120 µmol, 3.0 equiv.) and DIPEA (70 µL, 400 µmoL, 10.0 equiv.) in 1 mL of DMF was added to the resin and the mixture was stirred overnight at room temperature. Conjugation of the chelator was confirmed by analytical HPLC after cleavage and deprotection of a small amount of peptide. After completion of the reaction, the resin was washed with DMF (5 × 3 mL) and dichloromethane (3 × 3 mL). A solution of TFA/H₂O/triisopropylsilane (TIPS (v/v/v = 95:2.5:2.5) was added to the resin and the mixture was stirred 2 h at room temperature. The resin was removed from the solution by filtration, and ice-cold diethyl ether was added to the filtrate. The precipitate was purified by semi-preparative HPLC using **Condition 2** (see General section) to give 3 mg of peptide **2.** ESI-MS: m/z 1432.36 [M+H]⁺. The structure of (2) is given in Figure 1.

### Example 3 - DOTAM-4-Amcha-D-Phe-cyclo[Cys-Tyr-D-Trp-Lys-Thr-Cys]-Thr-OH (DOTAM-Amcha-TATE) (3):

4-(Fmoc-aminomethyl)cyclohexanecarboxylic acid (4-Amcha) (61 mg, 160 µmol, 4 equiv.) was dissolved in a solution of 0.38 M of HATU (400 µL, 152 µmol, 3.8 equiv.). After complete dissolution, a solution of 0.78 M of DIPEA (400 µL, 312 µmol, 7.8 equiv.) was added and the mixture was directly added to the resin **1** (40 µmol), prepared according to Example 1. The mixture was stirred 60 min at room temperature. The coupling was monitored by TNBS test and performed again when the test was negative. After the Fmoc deprotection, the coupling of DO3AM acetic acid and the global deprotection were performed following the same protocol described for Example 2. The peptide was purified by semi-preparative HPLC using **Condition 1** (see General section) to give 2 mg of the peptide **3.** ESI-MS: m/z 1571.36 [M+H]⁺. The structure of (3) is given in Figure 1.

### Example 4 - DOTAM-4-APipAc-D-Phe-cyclo[Cys-Tyr-D-Trp-Lys-Thr-Cys]-Thr-OH (DOTAM-Pip-TATE) (4):

The coupling of Fmoc-4-amino-1-carboxymethyl-piperidine (4-APipAc), DO3AM acetic acid and the global deprotection were performed following the same protocols described for **3.** The peptide was purified by semi-preparative HPLC using **Condition 1** (see section 1.2) to give the peptide **4.** ESI-MS: m/z 1572.62 [M+H]⁺.

### Example 5 - Preparation of [²¹²Pb]Pb-DOTAM-Amcha-TATE

²¹²PbCl₂ (100 kBq) in 0.1 M HCl was added to a mixture of DOTAM-Amcha-TATE (1 nmol), ascorbic acid/gentisic acid (10 pL, 50 mM), sodium acetate (30 µL, 2.5 M) and H₂O. The mixture was incubated for 20 min at r.t. The reaction was monitored by instant thin-layer chromatography (iTLC) on silica gel impregnated glass fiber sheets eluted with a solution of sodium citrate (0.1 M, pH 5.0). The reaction mixture was cooled down for 5 min and diethylenetriaminepentaacetic acid (DTPA) (5µL) was added to complex the remaining free lead-212. An aliquot was taken and injected in radio-HPLC.

### Example 6 - Preparation of [²⁰³Pb]Pb-DOTAM-Amcha-TATE

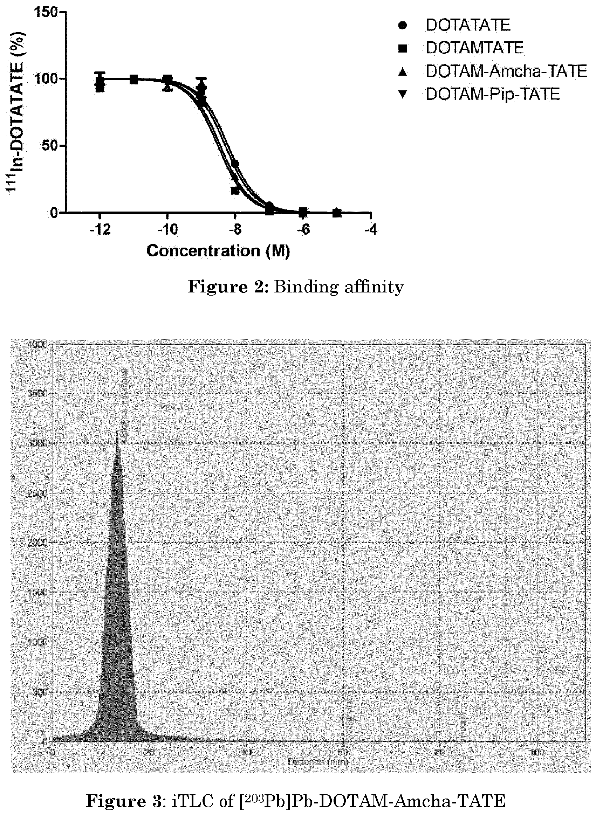

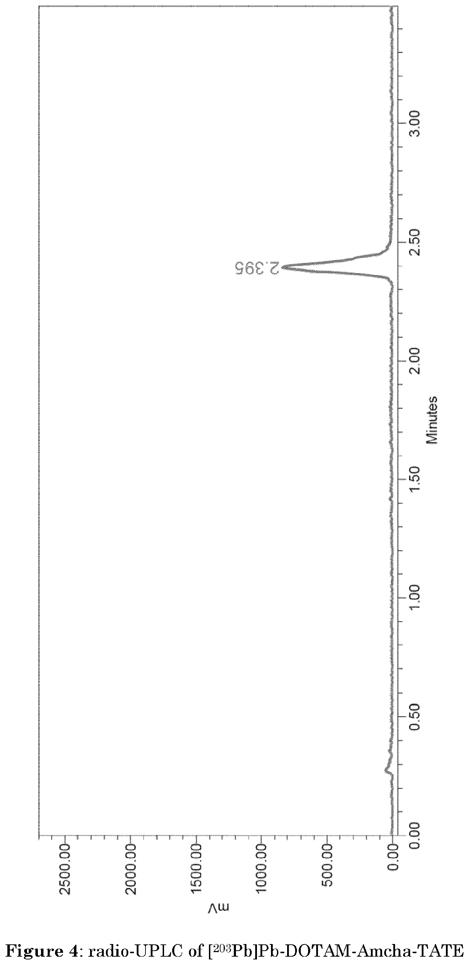

²⁰³PbCl₂ (40 MBq) in 0.1 M HCl was added to a mixture of DOTAM-Amcha-TATE (1 nmol), ascorbic acid/gentisic acid (10 pL, 50 mM), sodium acetate (30 µL, 2.5 M) and H₂O. The mixture was incubated for 20 min at r.t. The reaction was monitored by instant thin-layer chromatography (iTLC) on silica gel impregnated glass fiber sheets eluted with a solution of sodium citrate (0.1 M, pH 5.0). The reaction mixture was cooled down for 5 min and diethylenetriaminepentaacetic acid (DTPA) (5µL) was added to complex the remaining free lead-203. An aliquot was taken and injected in radio-HPLC. Results are shown in Figures 3 and 4.

### Example 7- In vitro and in vivo tests

### Binding affinity:

The binding affinity of the compounds to sstr2 was determined using a membrane-based competition binding assay. DOTATATE was used as a known positive control for affinity determination. Purified Chinese hamster ovary-Kl (CHO-K1) membranes overexpressing human sstr2 were incubated with [¹¹¹In]In-DOTATATE and competing non-radioactive ligand in a 96-well, 1.2 µm glass fibre filter plate (EMD Millipore, Darmstadt, Germany). Prior to assay, the plate filters were pre-soaked in 0.1% polyethyleneimine for 1h at ambient temperature. Following preincubation, membranes (25 µg/well, from a stock of 1.5 ug/ul or 400 U), [¹¹¹In]In-DOTATATE (0.05 nM) and various concentrations of competing peptides (10 µM to 1 pM) were diluted in assay buffer (25 mM HEPES, pH 7.4, 10 mM MgCl₂, 1 mM CaCl₂, 0.5% BSA) and incubated for 1 h at 27 °C with moderate shaking. Once complete, the incubation mixture was aspirated through the filters, followed by 6 washes with 200 µL ice-cold wash buffer (50 mM Tris-HCl pH 7.4, 0.2% BSA). Each filter was removed and counted on a PerkinElmer WIZARD 2480 gamma counter. The inhibition constant (Ki) was calculated by fitting the data to a onesite Fit-Ki curve in GraphPad Prism v7.02. Results are shown in Figure 2.

### Animal models:

Six-week-old male Balb/c nu/nu-specific and opportunistic pathogen-tree (SOPF) mice (Janvier Labs, Le Genest-Saint-Isle, France) were housed in individually ventilated cages, with 4 mice per cage. Upon arrival, mice were acclimated for 1 week, with access to food and water ad libitum. Mice were subcutaneously inoculated on the right shoulder with NCI-H69 cells (5 × 10⁶ cells suspended in 100 µL of 1/3 Matrigel (Corning Inc., Corning, NY, USA) and 2/3 Hank's balanced salt solution (Gibco). NCI-H69 xenografts were allowed to grow for 3 weeks. Tumor sizes were 391 ± 173 mm³ at the start of the studies. All animal experiments were approved by the Animal Welfare Committee of the Erasmus MC and were conducted in agreement with institutional guidelines (license number: AVD101002017867).

### In vivo single photon emission computed topology/computer tomography

### (SPECT/CT) and ex-vivo biodistribution with lead-203:

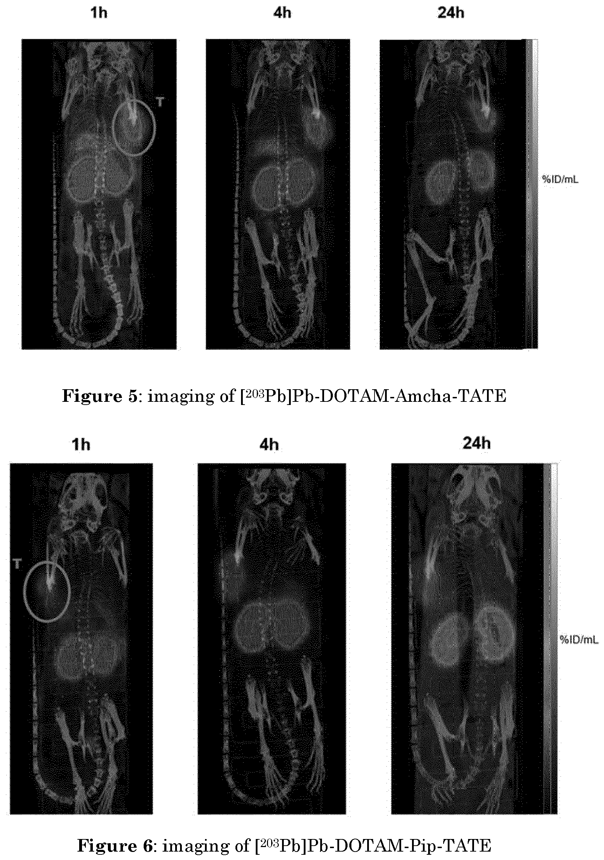

Mice (n = 4 per compound) were intravenously injected in the tail vein with 200 µL of Kolliphor^{®} HS 15 (Merck, Haarlerbergweg, The Netherlands) in PBS (0.06 mg/mL) containing [²⁰³Pb]Pb-DOTAM-Ameha-TATE (12.31 ± 10.25 MBq, 0.5 nmol) or [²⁰³Pb]Pb-DOTAM-Pip-TATE (13.06 ± 1.25 MBq, 0.5 nmol). At 1, 4 and 24 h post-injection (p.i.), mice were imaged in a prone position on a heated bed under 2% isoflurane/O₂ anesthesia, in a dedicated small-animal PET/SPECT/CT scanner (VECTor5CT scanner, MILabs B.V., Utrecht, The Netherlands) with a high sensitivity pinhole collimator (HE-GP-M, 1.6 mm pinholes, resolution of 0.85 mm for SPECT and sensitivity of > 10,700 cps). Whole-body SPECT images (transaxial field of view (FOV) 54 mm) were acquired over 1 frame of 20 min (for 1 and 4 h time-point) and 2 frame of 20 min (for 24 h time-point) using a spiral scan in normal scan mode, in list-mode acquisition. This was followed by a whole-body CT scan within 2.5 min, with the following imaging settings: full angle scan, angle step 0.75 degrees, normal scan mode, 50 kV tube voltage, 0.21 mA tube current, 500 µm aluminum filter. Reconstruction of the SPECT images was performed using the similarity-regulated SROSEM method (MILabs Rec 11.00 soft-ware, MILabs B.V., Houten, The Netherlands) performing 5 iterations with a vozel size of 0.4 mm, using 72 keV ± 30% and 280 keV ± 8% energy windows for lead-203. Reconstructed volumes of SPECT scans were post-filtered with an isotropic 3-dimensional Gaussian filter of 1 mm full width, at half-maximum. The CT and registered, attenuation-corrected SPECT images were analyzed using PMOD (PMOD 3.9, Zurich, Switzerland) and quantification was performed by placing volumes of interest (VOIs) around the tumors and kidneys. An Eppendorf tube filled with a solution of lead-203 of a known activity was measured to determine the calibration factor. The total activity measured in the VOI was divided by the volume of all VOI pixel values and multiplied by the calibration factor to obtain the percentage of injected activity per volume unit (% IA/mL). The results for [²⁰³Pb]Pb-DOTAM-Amcha-TATE are shown in Figure 5. The results for [²⁰³Pb]Pb-DOTAM-Pip-TATE are shown in Figures 6, 8 and 9.

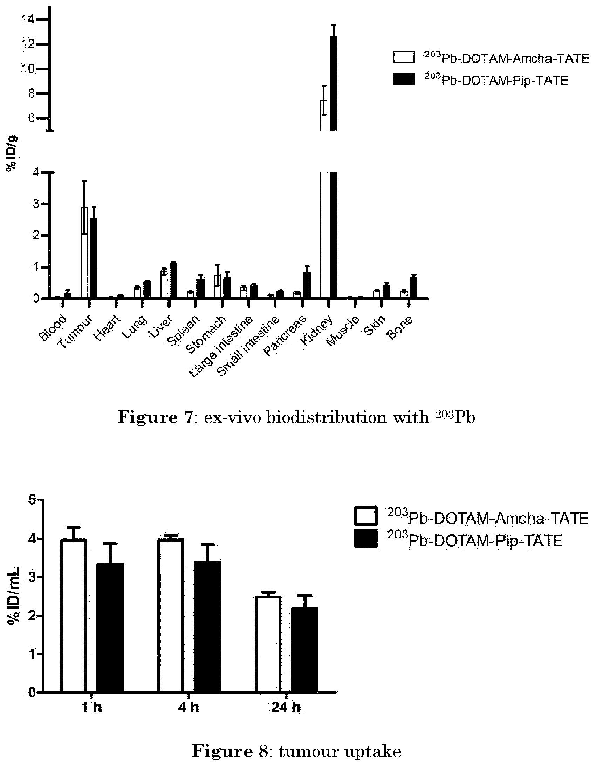

To determine the biodistribution of the compounds after imaging (1, 4 and 24 h p.i.), blood was collected via cardiac puncture under isoflurane/O₂ anesthesia, after which the mice were sacrificed. The tumor and organs of interest (prostate, pancreas, spleen, liver, GI tract (stomach, small intestine, cecum, large intestine), kidneys, lungs, heart, muscle, bone, and brain) were excised, washed in PBS and blotted dry. The stomach, intestines and cecum were emptied of their contents. The tumor was cut in half; one half was fresh-frozen for further analysis and the other half was collected for ex vivo optical imaging and radioactivity measurements The blood, tumor, and relevant organs were weighed and measured in a γ-counter. To determine the total injected radioactivity per animal, samples of the injected solutions were measured as well. The percentage of injected dose per gram (% ID/g) was determined for each tissue sample and corrected for both the injected volume and % ID present at the injection site (the tail). The results for [²⁰³Pb]Pb-DOTAM-Amcha-TATE and [²⁰³Pb]Pb-DOTAM-Pip-TATE are given in Figure 7.

### Biodistribution study with /ead-212:

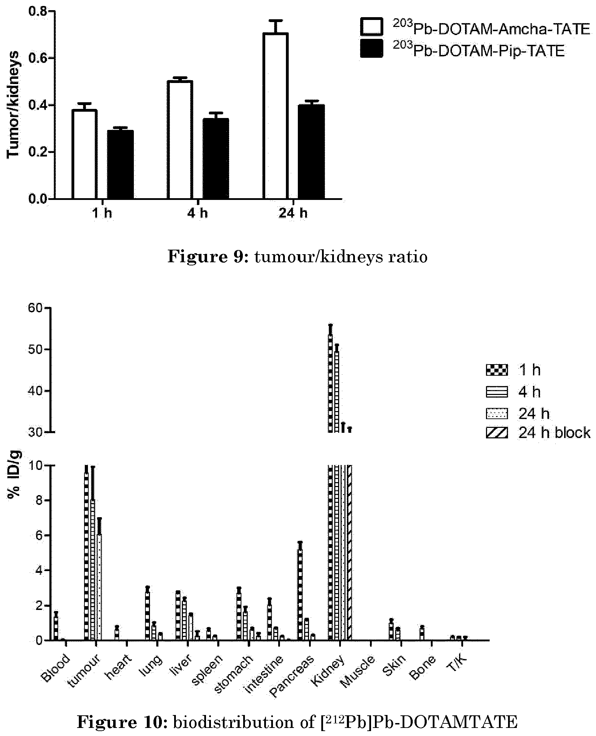

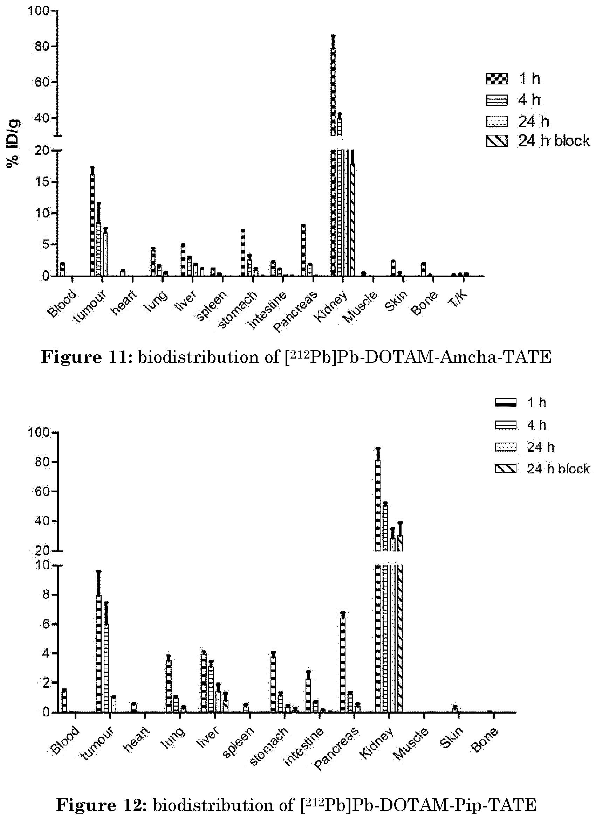

Biodistribution studies were performed to determine tumor and organ uptake of [²¹²Pb]Pb-DOTAMTATE, [²¹²Pb]Pb-DOTAM-Amcha-TATE or [²¹²Pb]Pb-DOTAM-Pip-TATE. Animals (n=4 for each time point and each compound) were injected intravenously with an average of 100 kBq/0.5 nmol at t=0. At 3 selected time points (1, 4 and 24 h) p.i. blood was collected via cardiac puncture under isoflurane/O₂ anesthesia, after which the mice were sacrificed. The tumor and organs of interest (prostate, pancreas, spleen, liver, GI tract (stomach, small intestine, large intestine), kidneys, lungs, heart, muscle, bone and tail) were excised, washed in PBS and blotted dry. To confirm receptor specificity of the 3 radiotracers uptake, NCI-H69-xenografted mice were co-injected with [²¹²Pb]Pb-DOTAMTATE, [²¹²Pb]Pb-DOTAM-Amcha-TATE or [²¹²Pb]Pb-DOTAM-Pip-TATE plus an excess (25 nmol) of the respective unlabelled peptide (n=3), after which tumor and organ uptake was determined at 24 h p.i. The blood, tumor, and relevant organs were weighed and measured in a γ-counter. To determine the total injected radioactivity per animal, calibration curve with lead-212 was determined. The percentage of injected dose per gram (% ID/g) was determined for each tissue sample and corrected for both the injected volume and % ID present at the injection site (the tail). The results for [²¹²Pb]Pb-DOTAM-Amcha-TATE and [²¹²Pb]Pb-DOTAM-Pip-TATE are given in Figure 11-12. Figure 10 shows the comparative biodistribution of [²¹²Pb]Pb-DOTAMTATE.

## Claims

1. A pharmaceutical compound, or a pharmaceutically acceptable salt thereof, according to formula (I)
Ch(M)-L-T (I)
wherein Ch represents a radioisotope chelator;
M represents a radioisotope;
T represents a sstr2-agonist;
L represents a linker comprising a moiety having a structure according to any of formulae IIa and IIb 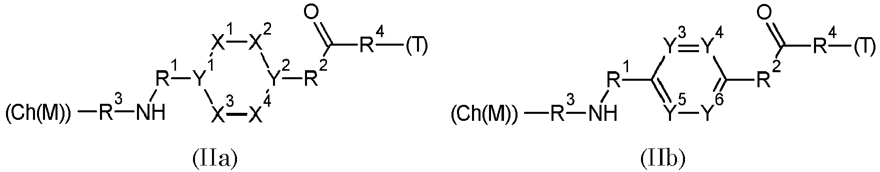 wherein
X¹-X⁴ are independently selected from C, O and N, optionally substituted by one or more heteroatoms;
Y¹-Y⁶ are independently selected from N and C, optionally substituted by one or more heteroatoms;
R¹ and R² are independently selected from the group consisting of a bond and branched and linear hydrocarbylenes, optionally substituted and/or interrupted by one or more heteroatoms;
R³ and R⁴ are independently selected from the group consisting of a bond and spacers.

2. The pharmaceutical compound, or a pharmaceutically acceptable salt thereof, according to claim 1, wherein R¹ and R² are independently selected from the group consisting of a bond and branched and linear (C₁-C₁₀)-alkylenes, optionally substituted and/or interrupted by one or more heteroatoms, preferably a bond and branched and linear (C₁-C₄)-alkylenes;
and wherein R³ and R⁴ are preferably both a bond.

3. The pharmaceutical compound, or a pharmaceutically acceptable salt thereof, according to any of the previous claims, wherein the linker L has a structure according to any of formulae IIaa, IIab, IIba and IIbb 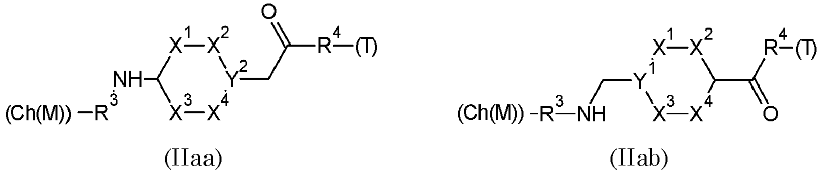 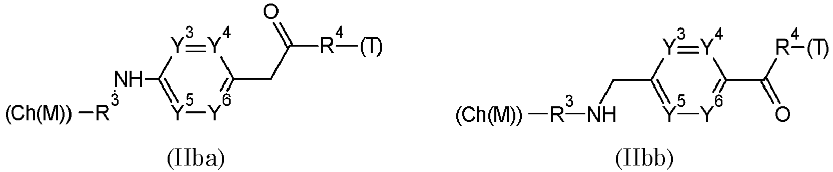 wherein
X¹-X⁴ are independently selected from C, O and N, optionally substituted by one or more heteroatoms, preferably wherein
Y¹-Y⁶ are independently selected from N and C, optionally substituted by one or more heteroatoms; preferably wherein one of Y¹-Y⁴ is N and the others are C, optionally substituted by one or more heteroatoms;
R³ and R⁴ are independently selected from the group consisting of a bond and spacers, preferably wherein R³ and R⁴ are both a bond;
wherein preferably for formulae IIaa and IIab, one of X¹-X⁴ and Y¹-Y² is selected from the O and N and the others are C, optionally substituted by one or more heteroatoms; and for formulae IIba and IIbb, one of Y³-Y⁶ is selected from C and N and the others are C, optionally substituted by one or more heteroatoms.

4. The pharmaceutical compound, or a pharmaceutically acceptable salt thereof, according to any of the previous claims, wherein the linker L has a structure according to any of formulae IIc-IIl 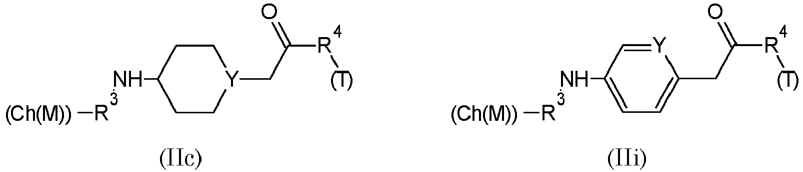 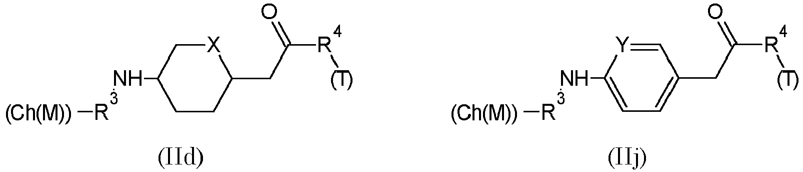 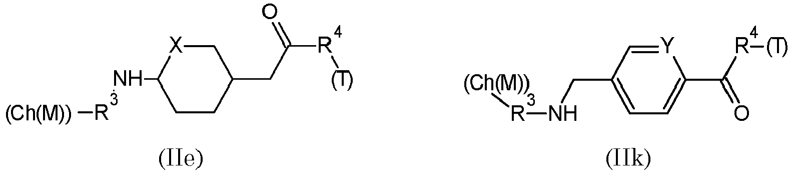 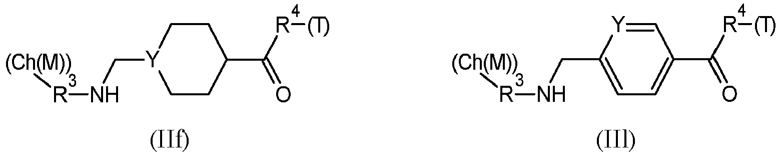 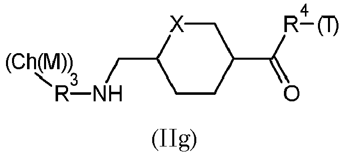 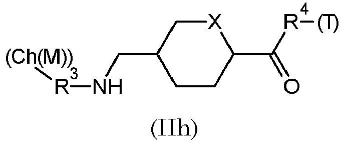 wherein
X is selected from the group consisting of C, N, and O, preferably N and C, more preferably C;
Y is selected from the group consisting of C and N, more preferably C; and
R³ and R⁴ are independently selected from the group consisting of a bond and spacers, preferably wherein R³ and R⁴ are both a bond.

5. The pharmaceutical compound, or a pharmaceutically acceptable salt thereof, according to any of the previous claims, wherein the linker L has a structure according to any of formulae IIca and IIfa, 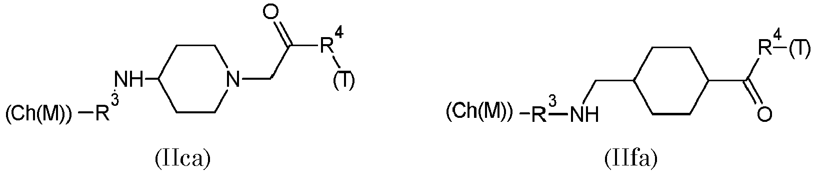 wherein R³ and R⁴ are independently selected from the group consisting of a bond and spacers, preferably wherein R³ and R⁴ are both a bond.

6. The pharmaceutical compound, or a pharmaceutically acceptable salt thereof, according to any of the previous claims, wherein the spacers that are independently represented by R³ and R⁴ are selected from the groups consisting of aliphatic spacers and peptide spacers, preferably from the group consisting of the peptide spacer (Xaa)₁₋₄, wherein each Xaa is independently a proteinogenic or non-proteinogenic amino acid residue, preferably wherein each Xaa is independently selected from the group consisting of D-amino acids of proteinogenic amino acids, *N^{ε}*,*N^{ε}*,*N^{ε}*-trimethyl-lysine, 2,3-diaminopropionic acid (Dap), 2,4-diaminobutyric acid (Dab), ornithine (Orn), homoarginine (hArg), 2-amino-4-guanidinobutyric acid (Agb),2-amino-3-guanidinopropionic acid (Agp), β-alanine, 4-aminobutyric acid, 5-aminovaleric acid, 6-aminohexanoic acid, 7-aminoheptanoic acid, 8-aminooctanoic acid, 9-aminononanoic acid, 10-aminodecanoic acid, 2-aminooctanoic acid, 2-aminoadipic acid ( 2-Aad), 3-aminoadipic acid (3-Aad), cysteic acid, diglycolic acid, NH₂(CH₂)₂O(CH₂)₂C(O)OH, NH₂(CH₂)₂[O(CH₂)₂]₂C(O)OH (dPEG2), NH₂(CH₂)₂[O(CH₂)₂]₃C(O)OH and NH₂(CH₂)₂[O(CH₂)₂]₄C(O)OH.

7. The pharmaceutical compound, or a pharmaceutically acceptable salt thereof, according to any of the previous claims, wherein the linker L has a structure according to any of formulae IIm and IIn.

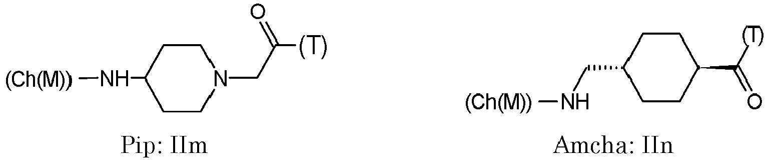

8. The pharmaceutical compound, or a pharmaceutically acceptable salt thereof, according to any of the previous claims, wherein the sstr2-agonist T is selected from the group consisting of octreotide, Tyr³-octreotide (TOC), Tyr³-octreotate (TATE), 1-Nal³-octreotide (NOC) and derivatives thereof, preferably wherein the sstr2-agonist is selected from the group consisting of octreotide and octreotate, most preferably wherein the sstr2-agonist is octreotate, or a pharmaceutically acceptable salt thereof.

9. The pharmaceutical compound, or a pharmaceutically acceptable salt thereof, according to any of the previous claims, wherein the sstr2-agonist T has a structure according to formula (III), 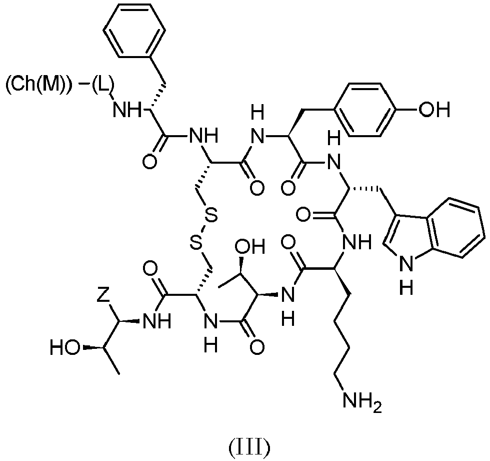 wherein Z is selected from the groups consisting of -CO₂H and -CH₂-OH.

10. The pharmaceutical compound, or a pharmaceutically acceptable salt thereof, according to any of the previous claims, wherein the radioisotope chelator Ch comprises a cyclic or branched polyaminopolycarboxylic moiety or amide derivative thereof, preferably wherein the radioisotope chelator Ch is selected from the group consisting of DOTA, PSC, DO3A, DOTAGA, DO3AM, DOTAM, pSCN-Bn-DOTA, p-SCN-Bn-TCMC, NOTA, NODAGA, NODASA, CB-DO2A, 3p-C-DEPA, TCMC, DTPA, CHX-A"-DTPA, 1B4M-DTPA, TETA, C-NETA, 3p-C-NETA, NE3TA, C-NE3TA, CB-TE2A, NETA, H₂azapa, H₂dedpa, H₄octapa, H₄py4pa, H₄Pypa, H₆phospha, H₄CHXoctapa, H₅decapa, H₄neunpa-p-Bn-NO₂, SHBED, HBED, H2-MACROPA, PCTA, Me-3,2-HOPO, CB-TE1A1P, CB-TE2P, MM-TE2A, DM-TE2A; sarcophagine and sarcophagine derivatives SarAr, diamSar, AmBaSar, and BaBaSar, TRAP, NOPO, AAZTA, DATA, CP256, YM103, PCTA, BCPA, DFO, trithiol chelates, mercaptoacetyl, hydrazinonicotinamide, dimercaptosuccinic acid, 1,2-ethylenediylbisL-cysteine diethyl ester, methylenediphosphonate, hexamethylpropyleneamineoxime, hexakis(methoxy isobutyl isonitrile), and analogues thereof, most preferably selected from the group consisting of DOTA, DO3AM and DOTAM.

11. The pharmaceutical compound, or a pharmaceutically acceptable salt thereof, according to any of the previous claims, wherein the radioisotope chelator Ch has a structure according to formula (IV) 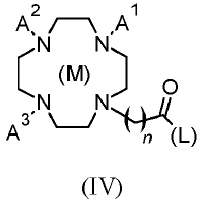 wherein n is 1-6, preferably 1,
A¹, A² and A³ are independently selected from the group consisting of H, alkyls, aliphatic acids, such as carboxylic acids, and amides and esters thereof, preferably from the group consisting of H, (C₁-C₆)-alkyl, (C₁-C₆)-alkylene-(CO₂A⁴), (C₁-C₆)-alkylene-(C(O)NA⁴A⁵), wherein A⁴ and A⁵ are independently selected from the group consisting of H and (C₁-C₆)-alkyls, preferably H, even more preferably wherein A¹, A² and A³ are all the same and selected from the group consisting of - CH₂CO₂H and -CH₂C(O)NH₂.

12. The pharmaceutical compound, or a pharmaceutically acceptable salt thereof, according to any of the previous claims, wherein the radioisotope M is selected from the group consisting of ²¹²Pb, ²⁰³Pb, ⁶⁴Cu, ⁶⁷Cu, ²¹²Bi, ⁶⁸Ga, ^{21S}Bi, ²²⁵Ac, ²⁴³Am, ²¹¹At, ²¹⁷At, ¹⁵⁴Dy, ¹⁴⁸Gd, ¹⁴⁶Sm, ¹⁴⁷Sm, ¹⁴⁹Tb, ¹⁵²Tb, ¹⁵⁵Tb, ¹⁶¹Tb, ¹⁶⁵Er, ⁷²As, ⁷⁷As, ⁴⁷Sc, ¹⁸⁸Re, ¹⁸⁶Re, ¹⁰⁵Rh, ¹⁰⁹Pd, ¹⁹⁹Au, ¹⁷⁵Yb, ¹⁴²Pr, ^{114*m*}In, ^{94*m*}Tc, ^{99*m*}Tc, ²²⁷Th, ²²⁹Th, ⁵⁹Fe, ⁶⁰Cu, ⁶¹Cu, ⁶²Cu, ⁶⁷Ga, ⁴⁴Sc, ⁸⁹Zr, ⁹⁰Nb, ⁸⁶Y, ⁹⁰Y, ¹¹¹In, ¹⁷⁷Lu, ^{117*m*}Sn, ¹⁵³Gd, ¹⁵³Sm, and ¹⁶⁶Ho, preferably from the group consisting of ⁶⁴Cu, ⁶⁷Ga, ⁶⁸Ga, ⁹⁰Y, ¹¹¹In, ¹⁷⁷Lu, ²¹²Pb and ²²⁵Ac, most preferably from the group consisting of ²¹²Pb and ¹⁷⁷Lu.

13. The pharmaceutical compound, or a pharmaceutically acceptable salt thereof, according to any of the previous claims, wherein having a structure according to any of formulae Ia and Ib, 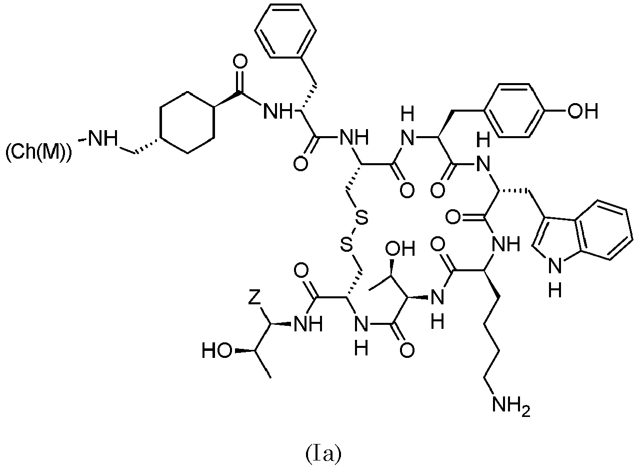 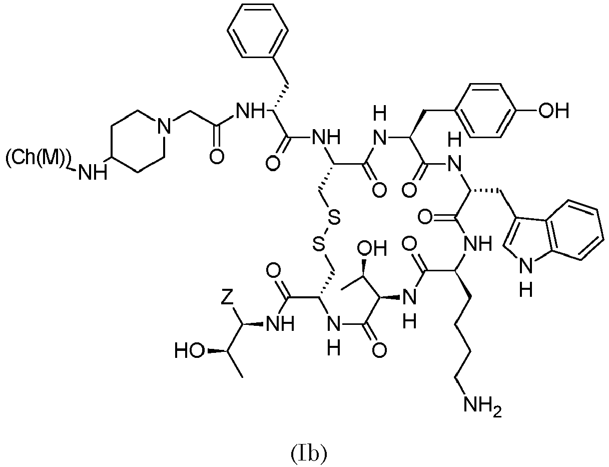 wherein Ch, M and Z are as defined in any of the previous claims.

14. The pharmaceutical compound, or a pharmaceutically acceptable salt thereof, according to claim 13, wherein said compound is of formula Ia, wherein Z is -CO₂H (such that the sstr2-agonist is TATE), Ch is DOTAM and M is ²¹²Pb (such that said compound can be abbreviated as [²¹²Pb]Pb-DOTAM-Amcha-TATE) or wherein Z is -CO₂H (such that the sstr2-agonist is TATE), Ch is DOTA and M is ¹⁷⁷Lu (such that said compound can be abbreviated as [¹⁷⁷Lu]Lu-DOTA-Ameha-TATE).

15. The pharmaceutical compound, or a pharmaceutically acceptable salt thereof, according to any of the previous claims, for use as a medicament, preferably for use in a medical treatment or diagnosis of tumors, in particular neuroendocrine tumors (NET).
